# EUROPEAN PATENT APPLICATION

(11) **EP 4 331 467 A1**
(43) Date of publication of application: **06.03.2024**
(21) Application number: 22795635.6
(22) Date of filing: 19.04.2022
(51) Int. Cl.: A61B 1/00, A61B 17/34

(54) **PUNCTURING DEVICE**

(30) Priority: 26.04.2021 JP 2021073757
(71) Applicant: ASAHI INTECC CO., LTD., Seto-shi, Aichi 489-0071 (JP)
(72) Inventor: YOSHIMATSU Daiki, Seto-shi, Aichi 489-0071 (JP); OSHIMA Fumiyoshi, Seto-shi, Aichi 489-0071 (JP)
(74) Representative: Prinz & Partner mbB
(86) International application number: PCT/JP2022/018174
(87) International publication number: WO 2022/230722

(57) **Abstract**

The puncturing device includes an inner portion, an outer peripheral portion, a protruding portion, and an imaging unit. The inner portion has a puncturing needle lumen that extends from a distal end to a proximal end. The outer peripheral portion extends along a central axis of the inner portion and surrounds the inner portion. The protruding portion is composed of a plurality of protruding pieces arranged around the central axis. Each of the plurality of protruding pieces is configured to be shiftable between a tapered state where a distal end of the protruding piece is closer to the central axis than a proximal end of the protruding piece, and a straight state where a distance from the distal end of the protruding piece to the central axis is equal to a distance from the proximal end of the protruding piece to the central axis.

## Description

### TECHNICAL FIELD

The technology disclosed herein relates to a puncturing device.

### BACKGROUND ART

A space between two pericardial sacs surrounding a heart is called a pericardium (or pericardial cavity) and is filled with a fluid called a pericardial fluid. Cardiac tamponade is a highly emergent disease in which a large amount of fluid accumulates in the pericardium for some reason, increasing intrapericardial pressure, which results in insufficient expansion and dysfunction of the heart as a pump, rapidly leading to a shock state. Cardiac tamponade is treated by pericardiocentesis to drain the pericardial fluid.

A puncturing device is used for pericardiocentesis (e.g. see Patent Literature 1) During the pericardiocentesis, the puncturing device is delivered from a patient's body surface to the pericardium in a visual field from an endoscope, and the pericardium is punctured using the puncturing device, .

### CITATION LIST

### Patent Literature

Patent Literature 1: JP 2016-158804 A

### SUMMARY OF INVENTION

### TECHNICAL PROBLEM

Since conventional puncturing devices have a flat distal end surface, the puncturing devices receive a high resistance from obstacles such as fat when delivered from a patient's body surface to a pericardium. When a conventional puncturing device is used, the visual field may be blocked by a forward obstacle in the advancing direction. Thus, the conventional puncturing devices have a problem that a procedure using a puncturing device cannot be efficiently executed. Such a problem is not limited to the puncturing devices for pericardiocentesis but is common to puncturing devices for puncturing a target tissue in a body.

A technique capable of solving the aforementioned problems is disclosed herein.

### SOLUTION TO PROBLEM

The technology disclosed herein can be implemented e.g. as the following aspects.
(1) The puncturing device disclosed herein includes an inner portion, an outer peripheral portion, a protruding portion, and an imaging unit. The inner portion has a puncturing needle lumen that extends from a distal end to a proximal end. The outer peripheral portion extends along a central axis of the inner portion and surrounds the inner portion. The protruding portion is a hollow part that protrudes from a distal end of the outer peripheral portion toward the distal end side. The imaging unit has an imaging element disposed on the distal end of the inner portion. The protruding portion is composed of a plurality of protruding pieces arranged around the central axis. Each of the plurality of protruding pieces is configured to be shiftable between a tapered state where the distal end of the protruding piece is closer to the central axis than the proximal end of the protruding piece, and a straight state where a distance from the distal end of the protruding piece to the central axis is equal to a distance from the proximal end of the protruding piece to the central axis.
   In the puncturing device, the hollow protruding portion that protrudes from the distal end of the outer peripheral portion toward the distal end side is composed of the plurality of protruding pieces arranged around the central axis, and each of the plurality of protruding pieces is configured to be shiftable between the tapered state and the straight state. Thus, when each protruding piece is shifted to the tapered state during delivery of the puncturing device, the distal end portion of the puncturing device can be rendered into a shape with a diameter gradually decreasing toward the distal end side to reduce a resistance received from obstacles such as fat. During the delivery of the puncturing device, each protruding piece is shifted from the tapered state to the straight state and then returned to the tapered state, thereby obstacles can be pushed aside from the front of the puncturing device by movement of each protruding piece, and a resistance from the obstacles accompanying the advance of the puncturing device can be effectively reduced. Furthermore, when each protruding piece is shifted to the straight state, a good visual field can be obtained from the imaging unit. Consequently, this puncturing device makes it possible to efficiently execute the procedure using the puncturing device.
(2) The puncturing device according to the above aspect may be configured such that first wire lumens that are each disposed corresponding to each of the plurality of protruding pieces and extend from the distal end to the proximal end of the outer peripheral portion are formed on the outer peripheral portion, second wire lumens that each communicate with each first wire lumen on the proximal end of the protruding piece and extend to the distal end portion of the protruding piece are formed on each of the plurality of protruding pieces, the puncturing device further includes operating wires that are each accommodated in each combination of the first wire lumen and the second wire lumen communicating with each other and have a distal end fixed to the distal end portion of the corresponding protruding piece, and each of the plurality of protruding pieces is tapered in its natural state and is configured to be shifted from the tapered state to the straight state when the operating wire is pulled toward the proximal end side. According to this puncturing device, each of the plurality of protruding pieces constituting the protruding portion can be shifted from the tapered state to the straight state by a simple operation of pulling each operating wire toward the proximal end side, and therefore the procedure using the puncturing device can be more efficiently executed.
(3) The puncturing device according to the above aspects may be configured such that the inner portion is composed of a shaft member having the puncturing needle lumen, the outer peripheral portion is composed of a sheath base portion as a part on a proximal end side of a tubular sheath that accommodates the shaft member, and the protruding portion is composed of a sheath protruding portion that protrudes from the distal end of the sheath base portion in the sheath toward the distal end side. In this puncturing device, the inner portion having the puncturing needle lumen and the outer peripheral portion surrounding the inner portion are constructed as separate members, and therefore it is possible to improve the design flexibility including selection of materials for the respective portions of the puncturing device.
(4) The puncturing device may be configured such that the inner portion is composed of a shaft inner portion that is a part close to the central axis in the shaft member having the puncturing needle lumen, the outer peripheral portion is composed of a shaft outer peripheral portion surrounding the shaft inner portion of the shaft member, and the protruding portion is composed of a shaft protruding portion that protrudes from the distal end of the shaft outer peripheral portion of the shaft member toward the distal end side. In this puncturing device, the inner portion having the puncturing needle lumen and the outer peripheral portion surrounding the inner portion are constructed as an integral member, and therefore it is possible to facilitate the operation of the puncturing device and to more efficiently execute the procedure using the puncturing device.
(5) The puncturing device according to the above aspects may be configured so as to further include a puncturing needle that is slidably disposed in the puncturing needle lumen. This puncturing device makes it possible to efficiently execute the procedure using the puncturing device including the puncturing needle.

The technology disclosed herein can be achieved in various aspects, e.g. in an aspect such as a puncturing device, a medical device including the puncturing device, and a production method thereof.

### BRIEF DESCRIPTION OF DRAWINGS

FIG. 1 is an explanatory diagram schematically illustrating a configuration of a puncturing device 100 according to the first embodiment.
FIG. 2 is an explanatory diagram schematically illustrating a configuration of the puncturing device 100 according to the first embodiment.
FIG. 3 is an explanatory diagram schematically illustrating a configuration of the puncturing device 100 according to the first embodiment.
FIG. 4 is an explanatory diagram schematically illustrating a configuration of the puncturing device 100 according to the first embodiment.
FIG. 5 is an explanatory diagram schematically illustrating a configuration of the puncturing device 100 according to the first embodiment.
FIG. 6 is an explanatory diagram schematically illustrating a configuration of the puncturing device 100 according to the first embodiment.
FIG. 7 is an explanatory diagram schematically illustrating a configuration of the puncturing device 100 according to the first embodiment.
FIG. 8 is an explanatory diagram schematically illustrating a configuration of a puncturing device 100a according to the second embodiment.

### EMBODIMENTS OF THE INVENTION

### A. First Embodiment:

A-1. Configuration of Puncturing Device 100:

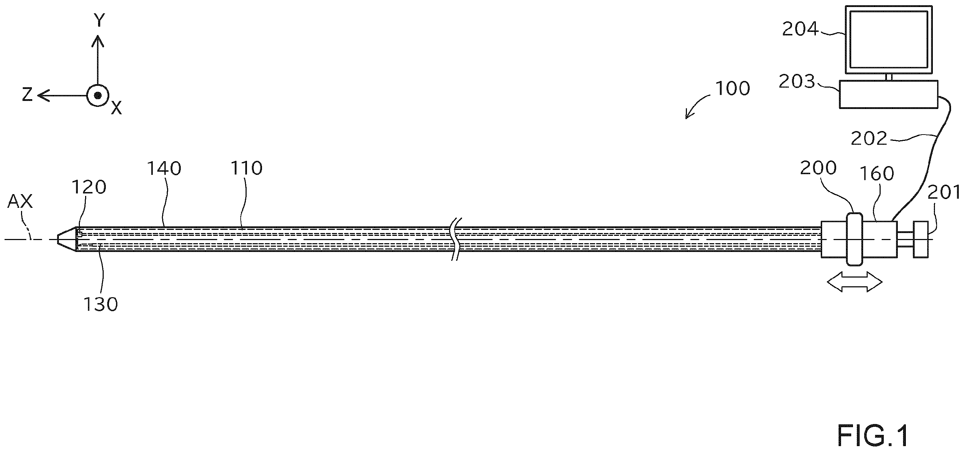

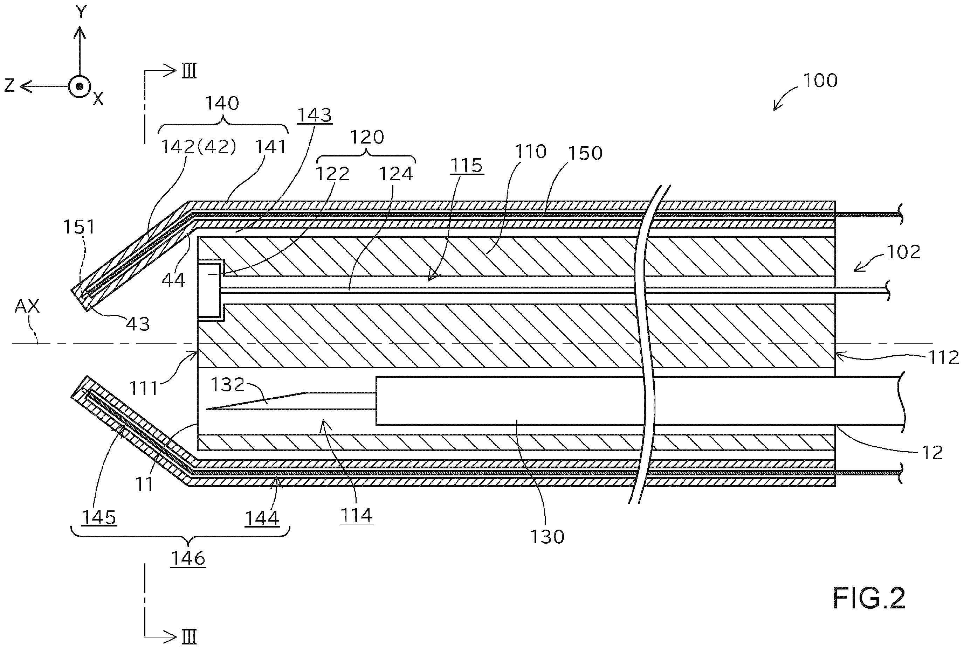

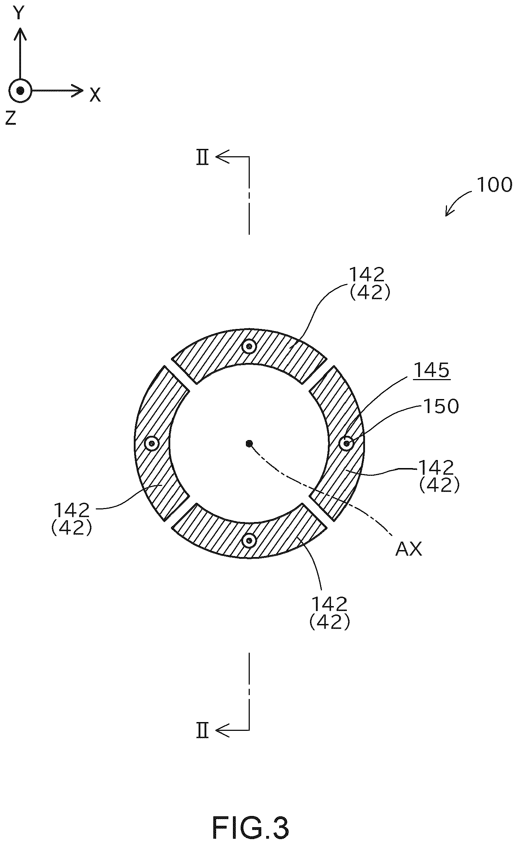

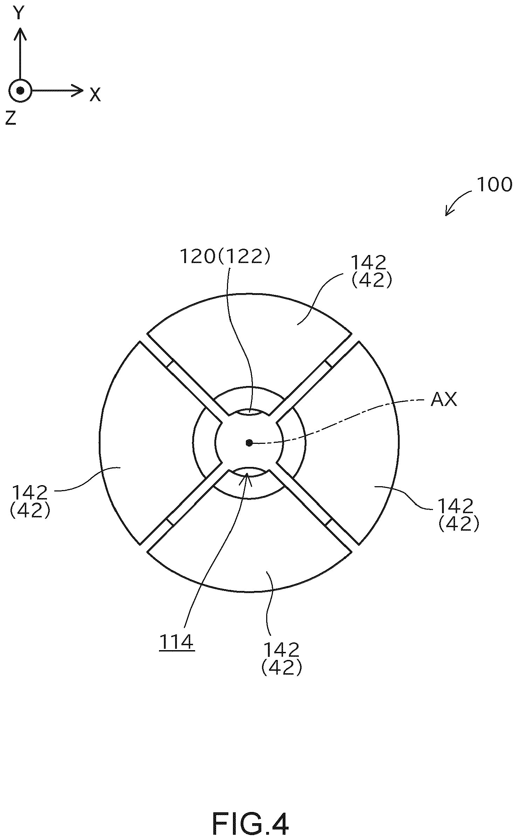

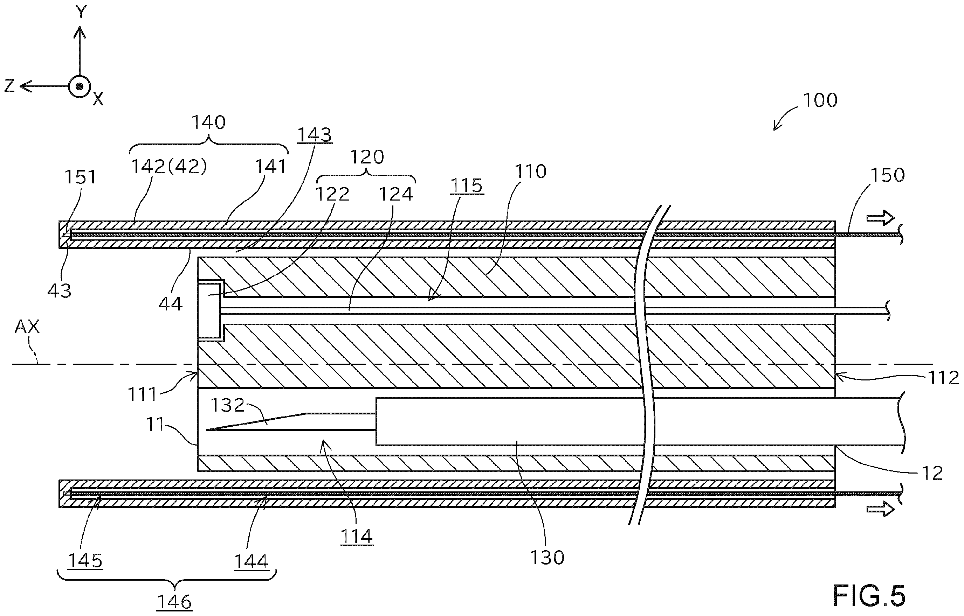

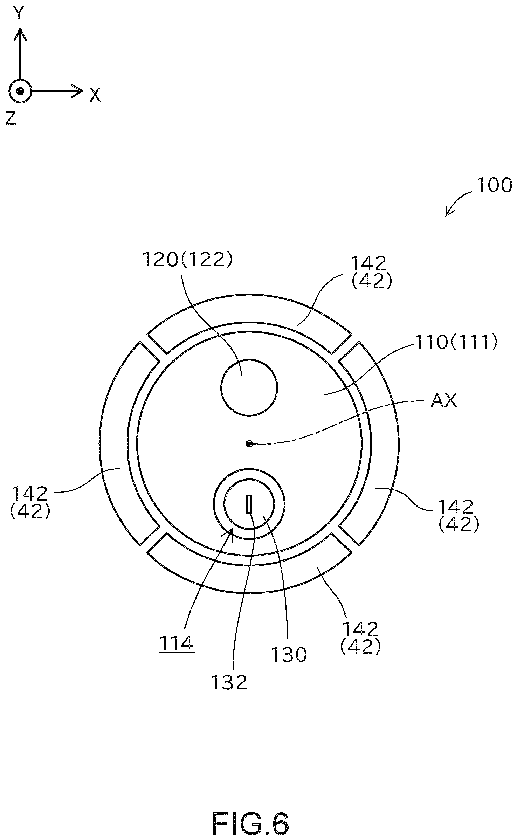

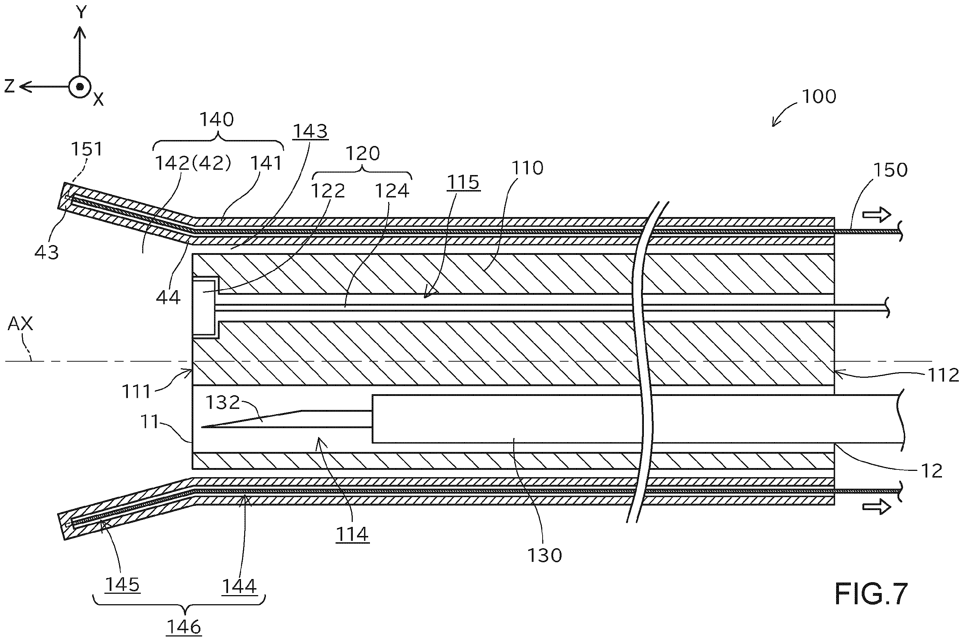

FIG. 1 to FIG. 7 are explanatory diagrams schematically illustrating a configuration of a puncturing device 100 according to the first embodiment. FIG. 1 illustrates a side configuration of the puncturing device 100 with each of below-mentioned protruding pieces 42 in a maximally tapered state. FIG. 2 illustrates a longitudinal sectional configuration of the puncturing device 100 with each of the below-mentioned protruding pieces 42 in a maximally tapered state (sectional configuration taken along II-II in FIG. 3). FIG. 3 illustrates a transverse sectional configuration of the puncturing device 100 in the same state (sectional configuration taken along III-III in FIG. 2). FIG. 4 illustrates a frontal configuration of the puncturing device 100 in the same state (configuration viewed from the positive side toward the negative side in Z-axis direction). FIG. 5 illustrates a longitudinal sectional configuration of the puncturing device 100 with each of the below-mentioned protruding pieces 42 in the straight state. FIG. 6 illustrates a frontal configuration of the puncturing device 100 in the same state. FIG. 7 illustrates a longitudinal sectional configuration of the puncturing device 100 with each of the below-mentioned protruding pieces 42 in a maximally reverse-tapered state. In some figures, a part of the puncturing device 100 (and components thereof) is omitted. In each figure, a positive side in Z-axis direction is a distal end side (far side) to be inserted into the body, and a negative side in Z-axis direction is a proximal end side (near side) to be manipulated by an operator such as a surgeon. Hereinafter, in the puncturing device 100 and the components thereof, an end on the distal end side is referred to as "distal end", the distal end and its vicinity are referred to as "distal end portion", an end on the proximal end side is referred to as "proximal end", and the proximal end and its vicinity are referred to as "proximal end portion".

The puncturing device 100 is a medical device that is inserted into a patient's body for pericardiocentesis. For example, the puncturing device 100 has a total length of about 400 mm to 600 mm and an outer diameter of about 1 mm to 5 mm. The distal end portion of the puncturing device 100 is a part that is inserted into a patient's body, and the proximal end portion of the puncturing device 100 is a portion that is gripped by an operator such as a surgeon.

The puncturing device 100 includes a shaft member 110, an imaging unit 120, a puncturing needle 130, a sheath 140, operating wires 150, and a connector 160.

The shaft member 110 is an elongated member. The shaft member 110 has e.g. an almost circular outer shape in a transverse section. The shaft member 110 is accommodated in a hollow portion 143 included in a sheath base portion 141 of the sheath 140 described later. The shaft member 110 is made of e.g. a resin or a metal. The shaft member 110 is an example of the inner portion in claims.

A needle ejection port 11 is formed on a distal end 111 of the shaft member 110, and a needle insertion port 12 is formed on the proximal end 112 of the shaft member 110. In the shaft member 110, a puncturing needle lumen 114 extending in the direction of central axis AX of the shaft member 110 is formed so as to communicate between the needle ejection port 11 and the needle insertion port 12. A transverse section of the puncturing needle lumen 114 has e.g. an almost circular outer shape. The puncturing needle 130 is slidably inserted into the puncturing needle lumen 114. The puncturing needle 130 is an elongated member having a sharp needle portion 132 on its distal end. The distal end portion of the puncturing needle 130 is located in the vicinity of the distal end portion of the puncturing needle lumen 114, and the proximal end portion of the puncturing needle 130 protrudes from the puncturing needle lumen 114 toward the proximal end side and is gripped by an operator.

The connector 160 is a hollow cylindrical member attached to the proximal end portion of the sheath base portion 141 that accommodates the shaft member 110. The connector 160 has an opening portion 201 on its proximal end side. The opening portion 201 communicates with the puncturing needle lumen 114 via the needle insertion port 12 of the shaft member 110 described later. The operator can insert the below-mentioned puncturing needle 130 through the opening portion 201 into the puncturing needle lumen 114. An almost annular operating portion 200 is attached to the outside of the connector 160. The operating portion 200 is configured to be slidable on the outside of the connector 160 toward the proximal end side or distal end side of the sheath base portion 141. The operating wires 150 described later are connected to the operating portion 200. Thus, when the operator moves the operating portion 200 toward the proximal end side of the sheath base portion 141, the operating wires 150 are pulled toward the proximal end side, and each of the protruding pieces 42 described later shifts from the maximally tapered state to the straight state or the reverse-tapered state. On the other hand, when the operating portion 200 which has been moved to the proximal end side is moved toward the distal end side of the sheath base portion 141 by the operator, a tension applied to the operating wires 150 toward the proximal end side is gradually released, and each protruding piece 42 shifts from the straight state or the reverse-tapered state to the maximally tapered state. The connector 160 is connected to a computer 203 via a cable 202. The cable 202 is electrically connected to a cable 124 that extends through an imaging unit lumen 115 described later via the connector 160. Since the cable 124 is electrically connected to the imaging unit 120 described later, the operator can control the imaging unit 120 by operating the computer 203. Since the computer 203 is also connected to a monitor 204, the operator can control the imaging unit 120 while confirming, on the monitor 204, an image captured by the imaging unit 120.

The shaft member 110 has the imaging unit lumen 115 that extends from the distal end 111 to the proximal end 112 in the direction of the central axis AX. The imaging unit 120 is disposed in the imaging unit lumen 115. More specifically, the imaging unit 120 has an imaging element 122 and a cable 124. The imaging element 122 is an image sensor (e.g. complementary metal-oxide (CMOS) image sensor, charge-coupled device (CCD) image sensor) that generates images through imaging. The imaging element 122 is disposed at a position of the imaging unit lumen 115 on the distal end 111 of the shaft member 110 such that an imaging face is directed to the distal end side. The cable 124 constitutes a transmission path for transmitting image signals generated by the imaging element 122 and extends through the imaging unit lumen 115 from the portion connected with the imaging element 122 toward the proximal end side. The proximal end portion of the cable 124 protrudes from the imaging unit lumen 115 toward the proximal end side and is connected, via the connector 160 and the cable 202, to the computer 203 that functionally serves as a controller for controlling the imaging unit 120. The imaging unit 120 may have a light source (e.g. light emitting diode (LED) and optical fiber) that emits light from the distal end of the shaft member 110 toward the distal end side.

The shaft member 110 may include, in addition to the puncturing needle lumen 114 and the imaging unit lumen 115, other lumens such as a combined device lumen for inserting a combined device such as forceps, a flush lumen for flushing a fluid, and a suction lumen for sucking some tissues.

The sheath 140 is an elongated member. The sheath 140 is made of e.g. a resin or a metal. The sheath 140 has a sheath base portion 141, a hollow sheath protruding portion 142 that protrudes from the distal end of the sheath base portion 141 toward the distal end side.

The sheath base portion 141 is an elongated tubular portion extending along the central axis AX of the shaft member 110. The sheath base portion 141 has an almost annular transverse sectional shape. The hollow portion 143 of the sheath base portion 141 accommodates the shaft member 110. In other words, the sheath base portion 141 surrounds the shaft member 110. In the direction of the central axis AX, the position of the distal end 111 of the shaft member 110 substantially coincides with the position of the distal end (portion connected with the sheath protruding portion 142) of the sheath base portion 141. The sheath base portion 141 and the shaft member 110 are connected to each other so that the shaft member 110 cannot move relative to the sheath base portion 141 in the Z-axis direction. In the first embodiment, the shaft member 110 is connected to the proximal end portion of the sheath base portion 141. However, the connection position therebetween can be changed arbitrarily. The sheath base portion 141 is an example of the outer peripheral portion in claims.

The sheath protruding portion 142 is composed of the plurality (four in the first embodiment) of protruding pieces 42 arranged around the central axis AX of the shaft member 110. Each protruding piece 42 is an almost trapezoidal plate-shaped piece having a proximal end 44 (portion connected with the sheath base portion 141) as a bottom side, and a distal end 43 as a top side. The sheath protruding portion 142 is an example of the protruding portion in the claims.

The connection portion between each protruding piece 42 and the sheath base portion 141 is more flexible than other portions of the sheath 140. To achieve such a flexible configuration, for example, a material and cross-sectional shape of the connection portion are made different from those of other portions. Thus, an inclination of each protruding piece 42 relative to the sheath base portion 141 (and the shaft member 110 accommodated in the sheath base portion 141) can be changed by pivoting the protruding piece 42 with the proximal end 44 of the protruding piece 42 as a fulcrum. Specifically, each protruding piece 42 is configured so as to be shiftable between a tapered state where the distal end 43 of the protruding piece 42 is closer to the central axis AX of the shaft member 110 than the proximal end 44 of the protruding piece 42 as illustrated in FIG. 1 to FIG. 4, and a straight state where the distance from the distal end 43 of the protruding piece 42 to the central axis AX is equal to the distance from the proximal end 44 of the protruding piece 42 to the central axis AX as illustrated in FIG. 5 and FIG. 6. Furthermore, in the first embodiment, each protruding piece 42 is also configured to be shiftable to a reverse-tapered state where the distal end 43 of the protruding piece 42 is farther from the central axis AX than the proximal end 44 of the protruding piece 42 as illustrated in FIG. 7.

In this embodiment, the protruding pieces 42 are not connected to each other and therefore can be independently pivoted. In the first embodiment, even when the distal end 43 of each protruding piece 42 is closest to the central axis AX (state illustrated in FIG. 1 to FIG. 4, hereinafter referred to as the "maximally tapered state"), the distal end 43 of each protruding piece 42 does not reach the central axis AX, resulting in a state where an opening is ensured around the center of the sheath protruding portion 142 composed of the respective protruding pieces 42 as viewed from the front of the puncturing device 100 (see FIG. 4). In the first embodiment, each protruding piece 42 is configured to be maximally tapered in its natural state (loads excluding a weight of the protruding piece 42 itself do not act on the protruding piece 42). Each figure illustrates one state of each protruding piece 42 as an example, in which the inclination of each protruding piece 42 can be continuously changed from the maximally tapered state to the maximally reverse-tapered state (where the distal end 43 of each protruding piece 42 is farthest from the central axis AX as illustrated in FIG. 7).

The sheath base portion 141 of the sheath 140 has a plurality (four in the first embodiment) of first wire lumens 144 that extend from the distal end (portion connected with each protruding piece 42) of the sheath base portion 141 to the proximal end. The protruding pieces 42 each have second wire lumens 145 that extend from the proximal end 44 of the protruding piece 42 to the distal end portion of the protruding piece 42. One first wire lumen 144 formed in the sheath base portion 141 communicates with the second wire lumen 145 formed in one protruding piece 42 to constitute one wire lumen 146 that extends from the proximal end of the sheath base portion 141 to the distal end portion of the sheath protruding portion 142. Similarly, another first wire lumen 144 formed in the sheath base portion 141 communicates with the second wire lumen 145 formed in another protruding piece 42 to constitute another wire lumen 146 that extends from the proximal end of the sheath base portion 141 to the distal end portion of the sheath protruding portion 142. In this way, the plurality of first wire lumens 144 formed in the sheath base portion 141 correspond to the plurality of protruding pieces 42.

Each wire lumen 146 accommodates the operating wire 150. The operating wire 150 is an elongated member made of e.g. a metal or a resin. The distal end portion 151 of the operating wire 150 is fixed to the distal end portion of the protruding piece 42, and the proximal end portion of the operating wire 150 protrudes from the proximal end of the wire lumen 146 toward the proximal end side. In the first embodiment, the distal end portion 151 of the operating wire 150 further extends from the distal end of the wire lumen 146 toward the distal end side and is embedded in the distal end portion of the protruding piece 42, so that the distal end portion 151 is fixed to the distal end portion of the protruding piece 42. Each protruding piece 42 is pivoted with the proximal end 44 of the protruding piece 42 as a fulcrum depending on a tension of the operating wires 150 caused by pulling the operating wires 150 toward the proximal end side to change the inclination of the protruding piece 42 relative to the sheath base portion 141 and shaft member 110, so that the protruding piece 42 becomes any one state from the maximally tapered state to the maximally reverse-tapered state.

A-2. Method for Using the Puncturing Device 100:
An example of a pericardiocentesis method using the puncturing device 100 will be explained below. First, the operator incises e.g. a lower part of a patient's sternum, insert the puncturing device 100 into the body through a chest hole formed by the incision, and deliver the distal end portion of the puncturing device 100 to the vicinity of the pericardium. At this time, the tension of each operating wire 150 of the puncturing device 100 is set to zero, and each protruding piece 42 is previously set to the maximally tapered state (see FIG. 1 to FIG. 4). As a result, the distal end portion of the puncturing device 100 has an almost conical shape with a diameter gradually decreasing toward the distal end side, and therefore a resistance received from obstacles such as fat can be reduced. If the puncturing device 100 receives a relatively high resistance when delivered, the operator pulls each operating wire 150 toward the proximal end side and then terminates the pulling of the operating wires 150. Thereby, the inclination of each protruding piece 42 of the puncturing device 100 is changed by pivoting the protruding piece 42 with the proximal end 44 of each protruding piece 42 as a fulcrum, so that the protruding piece shifts from the maximally tapered state to the straight state or the reverse-tapered state and then returns to the maximally tapered state. As a result, obstacles are pushed aside from the front of the puncturing device 100 by the movement of each protruding piece 42, and therefore the resistance accompanying the advance of the puncturing device 100 can be effectively reduced. When a visual field is ensured during the delivery of the puncturing device 100, the operator pulls each operating wire 150 toward the proximal end side to shift each protruding piece 42 to the straight state. Consequently, each protruding piece 42 can be prevented from occluding the front of the imaging unit 120 to obtain a good visual field from the imaging unit 120. The material and shape (thickness or the like) of each protruding piece 42 are set such that the protruding piece 42 has a rigidity sufficient for pushing away obstacles such as fat.

Once the distal end portion of the puncturing device 100 reaches the vicinity of the pericardium, the operator inserts the puncturing needle 130 into the puncturing needle lumen 114 from the proximal end (needle insertion port 12) of the puncturing needle lumen 114 formed in the shaft member 110 of the puncturing device 100. Subsequently, the operator grips and advances the proximal end portion of the puncturing needle 130 so that the distal end portion of the puncturing needle 130 reaches the vicinity of the distal end (needle ejection port 11) of the puncturing needle lumen 114. A puncturing needle 130 may be previously inserted into the puncturing needle lumen 114 prior to the delivery. Then, the operator pulls each operating wire 150 toward the proximal end side. Thereby, each protruding piece 42 of the puncturing device 100 shifts from the maximally tapered state to the straight state or the reverse-tapered state to obtain a good visual field from the imaging unit 120. Subsequently, the operator punctures the pericardium with the needle portion 132 of the puncturing needle 130 by advancing the puncturing needle 130 while confirming a puncture position from an image captured by the imaging unit 120. Prior to the puncture, the pericardial sac may be pulled using forceps (not illustrated) and held in this state.

After the puncture of the pericardial sac, the operator removes the puncturing needle 130 from the puncturing needle lumen 114 to the proximal end side. Then, a syringe (not illustrated) is attached to the proximal end side of the puncturing needle lumen 114, and the pericardial fluid is sucked using the syringe to drain the pericardial fluid outside the patient's body through the proximal end of the puncturing needle lumen 114. Subsequently, the puncturing device 100 is removed from the patient's body. Thus, the pericardiocentesis using the puncturing device 100 is completed.

A-3. Effect of First Embodiment:
As described above, the puncturing device 100 according to the first embodiment includes the shaft member 110, the sheath 140, and the imaging unit 120. The shaft member 110 has the puncturing needle lumen 114 that extends from the distal end 111 to the proximal end 112. The sheath 140 has a sheath base portion 141 and a sheath protruding portion 142. The sheath base portion 141 extends along the central axis AX of the shaft member 110 and surrounds the shaft member 110. The sheath protruding portion 142 is a hollow portion protruding from the distal end of sheath base portion 141 toward the distal end side. The imaging unit 120 has the imaging element 122 disposed on the distal end 111 of the shaft member 110. The sheath protruding portion 142 is composed of the plurality of protruding pieces 42 arranged around the central axis AX. Each of the plurality of protruding pieces 42 is configured to be shiftable between the tapered state where the distal end 43 of the protruding piece 42 is closer to the central axis AX than the proximal end 44 of the protruding piece 42, and the straight state where the distance from the distal end 43 of the protruding piece 42 to the central axis AX is equal to the distance from the proximal end 44 of the protruding piece 42 to the central axis AX.

As described above, in the puncturing device 100 according to the first embodiment, the hollow sheath protruding portion 142 that protrudes from the distal end of the sheath base portion 141 toward the distal end side is composed of the plurality of protruding pieces 42 arranged around the central axis AX, and each of the plurality of protruding pieces 42 is configured to be shiftable between the tapered state and the straight state. Thus, when each protruding piece 42 is shifted to the tapered state during the delivery of the puncturing device 100, the distal end portion of the puncturing device 100 can be rendered into a shape with a diameter gradually decreasing toward the distal end side to reduce a resistance received from obstacles such as fat. During the delivery of the puncturing device 100, each protruding piece 42 is shifted from the tapered state to the straight state and then returned to the tapered state, thereby obstacles can be pushed aside from the front of the puncturing device 100 by the movement of each protruding piece 42, and a resistance from the obstacles accompanying the advance of the puncturing device 100 can be effectively reduced. Furthermore, when each protruding piece 42 is shifted to the straight state, a good visual field can be obtained from the imaging unit 120. Consequently, the puncturing device 100 according to the first embodiment makes it possible to efficiently execute the procedure using the puncturing device 100.

In the puncturing device 100 according to the first embodiment, the sheath base portion 141 of the sheath 140 has the first wire lumens 144 that are each disposed corresponding to each of the plurality of protruding pieces 42 constituting the sheath protruding portion 142 and extend from the distal end to the proximal end of the sheath base portion 141. Each of the plurality of protruding pieces 42 has the second wire lumen 145 that communicates with the first wire lumen 144 on the proximal end 44 of the protruding piece 42 and extends to the distal end portion of the protruding piece 42. The puncturing device 100 further includes the operating wires 150 that are each accommodated in the wire lumen 146 as each combination of the first wire lumen 144 and the second wire lumen 145 communicating with each other. The distal end of each operating wire 150 is fixed to the distal end portion of the corresponding protruding piece 42. Each of the plurality of protruding pieces 42 is tapered in its natural state and is configured to be shifted from the tapered state to the straight state when the operating wire 150 is pulled toward the proximal end side. In the puncturing device 100 according to the first embodiment, each of the plurality of protruding pieces 42 constituting the sheath protruding portion 142 can be shifted from the tapered state to the straight state by a simple operation of pulling each operating wire 150 toward the proximal end side, and therefore the procedure using the puncturing device 100 can be executed more efficiently.

In the puncturing device 100 according to the first embodiment, the shaft member 110 having the puncturing needle lumen 114 and the sheath 140 (sheath base portion 141) surrounding the shaft member 110 are constructed as separate members, and therefore it is possible to improve the design flexibility including the selection of materials for each portion of the puncturing device 100.

B. Second Embodiment:

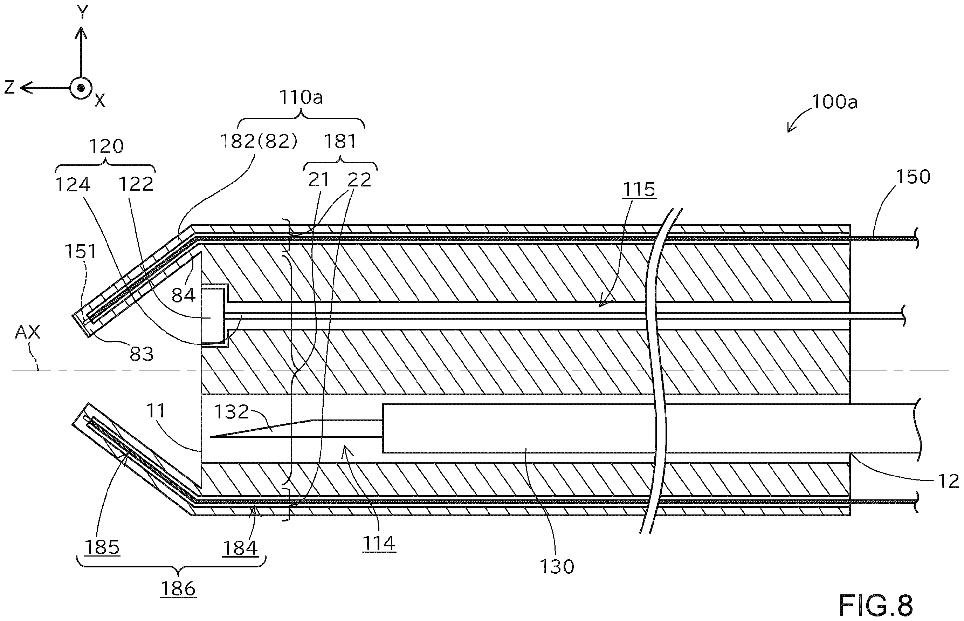

FIG. 8 is an explanatory diagram schematically illustrating a configuration of a puncturing device 100a according to the second embodiment. Hereinafter, components of the puncturing device 100a according to the second embodiment that are identical to the above-described components of the puncturing device 100 according to the first embodiment are provided with the same reference signs as in the first embodiment, thereby omitting the description thereof as appropriate.

In the puncturing device 100a according to the second embodiment, the shaft member 110 and the sheath 140 according to the first embodiment are constructed as an integral member (shaft member 110a).

The shaft member 110a has a shaft base portion 181 and a shaft protruding portion 182. The shaft base portion 181 has a shaft inner portion 21 that is a part close to the central axis AX, and a shaft outer peripheral portion 22 surrounding the shaft inner portion 21. The shaft inner portion 21 has the puncturing needle lumen 114, and the imaging element 122 of the imaging unit 120 is disposed on the distal end of the shaft inner portion 21.

The shaft protruding portion 182 protrudes from the distal end of the shaft outer peripheral portion 22 of the shaft base portion 181 toward the distal end side. Like the sheath protruding portion 142 according to the first embodiment, the shaft protruding portion 182 is composed of a plurality (four in the second embodiment) of protruding pieces 82 arranged around the central axis AX. An inclination of each protruding piece 82 relative to the shaft outer peripheral portion 22 and the shaft inner portion 21 can be changed by pivoting the protruding piece 82 with a proximal end 84 of the protruding piece 82 as a fulcrum. That means, each protruding piece 82 is configured so as to be shiftable between a tapered state where a distal end 83 of the protruding piece 82 is closer to the central axis AX than the proximal end 84 of the protruding piece 82, and a straight state where a distance from the distal end 83 of the protruding piece 82 to the central axis AX is equal to a distance from the proximal end 84 of the protruding piece 82 to the central axis AX. Furthermore, in the second embodiment, each protruding piece 82 is also configured to be shiftable to a reverse-tapered state where the distal end 83 of the protruding piece 82 is farther from the central axis AX than the proximal end 84 of the protruding piece 82.

The shaft outer peripheral portion 22 of the shaft base portion 181 has a plurality (four in the second embodiment) of first wire lumens 184, the protruding pieces 82 each have second wire lumens 185, and each first wire lumen 184 communicates with each second wire lumen 185 to constitute a plurality of wire lumens 186. Each wire lumen 186 accommodates the operating wire 150. The inclination of each protruding piece 82 is changed by pivoting the protruding piece 82 with the proximal end 84 of the protruding piece 82 as a fulcrum depending on a tension of the operating wire 150 caused by pulling the operating wire 150 toward the proximal end side, so that the protruding piece 82 becomes any state from the maximally tapered state to the maximally reverse-tapered state.

In the puncturing device 100a according to the second embodiment, the shaft inner portion 21 of the shaft base portion 181 is an example of the inner portion in the claims, the shaft outer peripheral portion 22 of the shaft base portion 181 is an example of the outer peripheral portion in the claims, and the shaft protruding portion 182 is an example of the protruding portion in the claims.

As described above, the puncturing device 100a according to the second embodiment includes the shaft inner portion 21 that has the puncturing needle lumen 114 extending from the distal end to the proximal end, the shaft outer peripheral portion 22 that extends along the central axis AX of the shaft inner portion 21 and surrounds the shaft inner portion 21, the hollow shaft protruding portion 182 that protrudes from the distal end of the shaft outer peripheral portion 22 toward the distal end side, and the imaging unit 120 having the imaging element 122 disposed on the distal end of the shaft inner portion 21. The shaft protruding portion 182 is composed of the plurality of protruding pieces 82 arranged around the central axis AX. Each of the plurality of protruding pieces 82 is configured so as to be shiftable between the tapered state where the distal end 83 of the protruding piece 82 is closer to the central axis AX than the proximal end 84 of the protruding piece 82, and the straight state where the distance from the distal end 83 of the protruding piece 82 to the central axis AX is equal to the distance from the proximal end 84 of the protruding piece 82 to the central axis AX. Therefore, according to the puncturing device 100a according to the second embodiment, the procedure using the puncturing device 100a can be efficiently executed like the puncturing device 100 according to the first embodiment.

In the puncturing device 100a according to the second embodiment, the shaft outer peripheral portion 22 of the shaft base portion 181 has the first wire lumens 184 that are each disposed corresponding to each of the plurality of protruding pieces 82 and extend from the distal end to the proximal end of the shaft outer peripheral portion 22, and the plurality of protruding pieces 82 each have the second wire lumens 185 that each communicate with each first wire lumen 184 on the proximal end 84 of the protruding piece 82 and extend to the distal end portion of the protruding piece 82. The puncturing device 100a further includes the operating wires 150 that are each accommodated in the wire lumen 186 as each combination of the first wire lumen 184 and the second wire lumen 185 communicating with each other. The distal end of each operating wire 150 is fixed to the distal end portion of the corresponding protruding piece 82. Each of the plurality of the protruding pieces 82 is tapered in its natural state and is configured to be shifted from the tapered state to the straight state when the operating wire 150 is pulled toward the proximal end side. Thus, in the puncturing device 100a according to the second embodiment, the procedure using the puncturing device 100a can be executed more efficiently like the puncturing device 100 according to the first embodiment.

In the puncturing device 100a according to the second embodiment, the shaft inner portion 21 having the puncturing needle lumen 114, the shaft outer peripheral portion 22 surrounding the shaft inner portion 21, and the shaft protruding portion 182 extending from the distal end of the shaft outer peripheral portion 22 to the distal end side are constructed as an integral member, and therefore it is possible to facilitate the operation of the puncturing device 100a and to execute the procedure using the puncturing device 100a more efficiently.

C. Modification Example:
The technology disclosed herein is not limited to the above-described embodiments and can be modified in various forms without departing from the gist of the disclosed embodiments. For example, the following modifications are possible.

The configurations of the puncturing device 100 according to the above embodiments are merely an example and may be modified in various ways. For example, in the above embodiments, the sheath protruding portion 142 (or shaft protruding portion 182, the same applies to the following) is composed of four protruding pieces 42 (or protruding pieces 82, the same applies to the following), but the sheath protruding portion 142 may be composed of two or more and three or less protruding pieces 42, or five or more protruding pieces 42.

In the above embodiments, each protruding piece 42 is configured to be shiftable among the tapered state, the straight state, and the reverse-tapered state, but each protruding piece 42 may be configured to be shiftable between the tapered state and the straight state but unshiftable to the reverse-tapered state.

In the above embodiments, even when each protruding piece 42 is in the maximally tapered state, the opening is ensured in the vicinity of the center of the sheath protruding portion 142 composed of each protruding piece 42 as viewed from the front of the puncturing device 100, but the puncturing device may be configured such that, when each protruding piece 42 is in the maximally tapered state, the vicinity of the center of the sheath protruding portion 142 is substantially closed.

In the above embodiments, the operating wires 150 are accommodated in the wire lumens 146, but the operating wires 150 may be accommodated in another portion (e.g. the hollow portion 143 of the shaft member 110). In the above embodiments, the pivoting of each protruding piece 42 with the proximal end as a fulcrum is achieved by the operating wires 150, but may be achieved by other means (e.g. a balloon disposed in the vicinity of the proximal end of each protruding piece 42).

In the above embodiments, each protruding piece 42 is configured to be maximally tapered in its natural state (loads excluding a weight of the protruding piece 42 itself do not act on the protruding piece 42), but the puncturing device does not necessarily have such a configuration. For example, each protruding piece 42 may be configured to be straight in its natural state.

In the above embodiments, each protruding piece 42 may be made of a material that transmits at least light of a specific wavelength used for imaging so that imaging using the imaging unit 120 is possible even when each protruding piece 42 is in the tapered state.

In the above embodiments, as the method for fixing the distal end portion 151 of the operating wire 150 to the distal end portion of each protruding piece 42, another method may be adopted (e.g. a method in which the distal end portion 151 of the operating wire 150 is made to protrude from the distal end of the protruding piece 42 toward the distal end side, and the distal end portion 151 is folded to the inner peripheral side and fixed to the inner peripheral surface of the protruding piece 42).

The methods for using the puncturing device 100 according to the above embodiments are merely examples, and the puncturing device 100 may be used by another method.

Although the puncturing device 100 has been explained in the above embodiments, the techniques disclosed herein can be applied not only to the puncturing device for pericardiocentesis but also to other puncturing devices for puncturing a target tissue in a body.

### DESCRIPTION OF REFERENCE NUMERALS

11: Needle ejection port
12: Needle insertion port
21: Shaft inner portion
22: Shaft outer peripheral portion
42: Protruding piece
43: Distal end
44: Proximal end
82: Protruding piece
83: Distal end
84: Proximal end
100: Puncturing device
110: Shaft member
111: Distal end
112: Proximal end
114: Puncturing needle lumen
115: Imaging unit lumen
120: Imaging unit
122: Imaging element
124: Cable
130: Puncturing needle
132: Needle portion
140: Sheath
141: Sheath base portion
142: Sheath protruding portion
143: Hollow portion
144: First wire lumen
145: Second wire lumen
146: Wire lumen
150: Operating wire
151: Distal end portion
160: Connector
181: Shaft base portion
182: Shaft protruding portion
184: First wire lumen
185: Second wire lumen
186: Wire lumen
200: Operating portion
201: Opening portion
202: Cable
203: Computer
204: Monitor
AX: Central axis

## Claims

1. A puncturing device, comprising:
an inner portion that has a puncturing needle lumen extending from a distal end to a proximal end;
an outer peripheral portion that extends along a central axis of the inner portion and surrounds the inner portion;
a hollow protruding portion that protrudes from a distal end of the outer peripheral portion toward a distal end side; and
an imaging unit that has an imaging element disposed on a distal end of the inner portion,
wherein the protruding portion is composed of a plurality of protruding pieces arranged around the central axis, and
each of the plurality of protruding pieces is configured to be shiftable between a tapered state where a distal end of the protruding piece is closer to the central axis than a proximal end of the protruding piece, and a straight state where a distance from the distal end of the protruding piece to the central axis is equal to a distance from the proximal end of the protruding piece to the central axis.

2. The puncturing device according to claim 1, wherein
first wire lumens that are each disposed corresponding to each of the plurality of protruding pieces and extend from the distal end to the proximal end of the outer peripheral portion are formed on the outer peripheral portion,
second wire lumens that each communicate with each first wire lumen on the proximal end of the protruding piece and extend to the distal end portion of the protruding piece are formed on each of the plurality of protruding pieces,
the puncturing device further comprises operating wires that are each accommodated in each combination of the first wire lumen and the second wire lumen communicating with each other and have a distal end fixed to the distal end portion of the corresponding protruding piece, and
each of the plurality of protruding pieces is tapered in its natural state and is configured to be shifted from the tapered state to the straight state when the operating wire is pulled toward a proximal end side.

3. The puncturing device according to claim 1 or 2, wherein
the inner portion is composed of a shaft member having the puncturing needle lumen,
the outer peripheral portion is composed of a sheath base portion as a part on a proximal end side of a tubular sheath that accommodates the shaft member, and
the protruding portion is composed of a sheath protruding portion that protrudes from a distal end of the sheath base portion in the sheath toward the distal end side.

4. The puncturing device according to claim 1 or 2, wherein
the inner portion is composed of a shaft inner portion that is a part close to the central axis in the shaft member having the puncturing needle lumen,
the outer peripheral portion is composed of a shaft outer peripheral portion surrounding the shaft inner portion of the shaft member, and
the protruding portion is composed of a shaft protruding portion that protrudes from a distal end of the shaft outer peripheral portion of the shaft member toward the distal end side.

5. The puncturing device according to any one of claims 1 to 4, further comprising a puncturing needle that is slidably disposed in the puncturing needle lumen.
